# EUROPEAN PATENT SPECIFICATION

(11) **EP 1 893 039 B1**
(45) Date of publication and mention of the grant of the patent: **30.05.2012**
(21) Application number: 06724215.6
(22) Date of filing: 11.04.2006
(51) Int. Cl.: A23L 1/30, A23L 1/29, A61K 31/202

(54) **METHOD FOR TREATMENT AND PREVENTION OF RESPIRATORY INSUFFICIENCY**
VERFAHREN ZUR BEHANDLUNG UND VERHINDERUNG VON RESPIRATORISCHER INSUFFIZIENZ
TRAITEMENT ET PREVENTION DE L'INSUFFISANCE RESPIRATOIRE

(30) Priority: 21.04.2005 EP 05008771
(43) Date of publication of application: 05.03.2008
(73) Proprietor: N.V. NUTRICIA, 2712 HM Zoetermeer (NL)
(72) Inventor: BEERMANN, Christopher, 61267 Neu-Anspach (DE); ZIELEN, Stefan, 65719 Hofheim (DE); BÖHLES, Hans, Josef, 60322 Frankfurt am Main (DE); SCHUBERT, Ralf, 64287 Darmstadt (DE)
(74) Representative: Köster, Hajo
(86) International application number: PCT/EP2006/003285
(87) International publication number: WO 2006/111295

(56) References cited:
- EP-A- 0 457 950
- EP-A- 0 711 503
- WO-A-93/19624
- MANCUSO P ET AL: "EFFECTS OF EICOSAPENTAENOIC AND GAMMA-LINOLENIC ACID ON LUNG PERMEABILITY AND ALVEOLAR MACROPHAGE EICOSANOID SYNTHESIS IN ENDOTOXIC RATS" CRITICAL CARE MEDICINE, WILLIAMS AND WILKINGS COMPANY, BALTIMORE, MA, US, vol. 25, no. 3, March 1997 (1997-03), pages 523-532, XP008044589 ISSN: 0090-3493
- PALOMBO J D ET AL: "Effect of short-term enteral feeding with eicosapentaenoic and gamma-linolenic acids on alveolar macrophage eicosanoid synthesis and bactericidal function in rats." CRITICAL CARE MEDICINE. SEP 1999, vol. 27, no. 9, September 1999 (1999-09), pages 1908-1915, XP008053257 ISSN: 0090-3493
- GADEK J E ET AL: "Effect of enteral feeding with eicosapentaenoic acid, [gamma]-linolenic acid, and antioxidants in patients with acute respiratory distress syndrome" CRITICAL CARE MEDICINE 1999 UNITED STATES, vol. 27, no. 8, 1999, pages 1409-1420, XP008053256 ISSN: 0090-3493
- PEAT JENNIFER K ET AL: "Three-year outcomes of dietary fatty acid modification and house dust mite reduction in the Childhood Asthma Prevention Study." THE JOURNAL OF ALLERGY AND CLINICAL IMMUNOLOGY. OCT 2004, vol. 114, no. 4, October 2004 (2004-10), pages 807-813, XP002348290 ISSN: 0091-6749
- PATENT ABSTRACTS OF JAPAN vol. 1998, no. 01, 30 January 1998 (1998-01-30) & JP 09 234019 A (NOF CORP), 9 September 1997 (1997-09-09)

## Description

### FIELD OF THE INVENTION

The present invention relates to a method for the prevention and treatment of dust mite allergy.

### BACKGROUND OF THE INVENTION

House mite (*Dermatophagoides*) allergy is a hypersensitive reaction to proteins present in the excretion of dust mites and can result in respiratory insufficiencies such as airway obstruction and bronchial hyperreactivity. Such hypersensitive reaction is called "dust mite induced respiratory insufficiency" in the present documents.

House dust mites are found in almost all homes. The excretion of dust mites can form a serious threat to those sensitive to said excretion or to critically ill patients. The dust mites are not visible to the unaided eye due to their very small size (250 to 300 microns in length) and translucent bodies, and thus difficult to detect in the house.

In the study by Mihrishahi et al (J Allergy Clin Immunol, 2003; 111:162-8) no benefit for the active house dust mite avoidance intervention on symptoms was found. Presently an important treatment of dust mite induced respiratory insufficiency is symptom prevention by the administration of e.g. bronchiodilators.

It has been suggested that including more fish oil in the diet has a beneficial effect on wheezing (Mihrshahi et al). However,

EP-A 457 950 describes a method for treatment of disorders of inflammatory origin by biosynthesis of leukotrienes with stearidonic acid.

WO-A 02 092 073 describes the production and use, and in particular, the extraction, separation, synthesis and recovery of polar lipid-rich fractions containing gamma linolenic acid and/or stearidonic acid from seeds and microorganisms and their use in human food applications, animal feed, pharmaceuticals and cosmetics.

US-A 5 223 285 describes a liquid nutritional product for enteral feeding containing a fat source which provides desirable effects when fed to pulmonary patients. The fat source having a weight ratio of [n-(6) to n-(3)] fatty acids from the group consisting of linoleic acid (18:2n6), gamma-linolenic acid (18:3n6), and arachidonic acid (20:4n6) to fatty acids from the group consisting of alpha-linolenic acid (18:3n3), stearidonic acid (18:4n3), eicosapentaenoic acid (20:5n3), docosapentaenoic acid (22:5n3) and docosahexaenoic acid (22:6n3) in the range of about 1.5 to about 3.0, a ratio of linoleic acid (18:2n6) to alpha-linolenic acid (18:3n3) in the range of about 3.0 to about 10.0, and a ratio of the sum of eicosapentaenoic acid (20:5n3) and docosahexaenoic acid (22:6n3) to gamma-linolenic acid (18:3n6) in the range of about 1.0 to about 10.0. In a preferred embodiment the nutritional product contains quantities of nutrients having anti-oxidant properties in vivo, such as beta-carotene, vitamin E, vitamin C, selenium, and taurine.

The following document "PEAT JENNIFER K ET AL: "Three-year outcomes of dietary fatty acid modification and house dust mite reduction in the Childhood Asthma Prevention Study." THE JOURNAL OF ALLERGY AND CLINICAL IMMUNOLOGY. OCT 2004, vol. 114, no. 4, October 2004 (2004-10), pages 807-813, XP002348290 ISSN: 0091-6749" describes the use of omega fatty acids from tuna oil for infants to prevent dust mite induced allergy. Tuna oil contains EPA, DHA and STA. The STA/DHA ratio is at least 0,1.

### SUMMARY OF THE INVENTION

The invention is best described by the claims 1 and 7

It has now surprisingly been found that a composition containing eicosapentaenoic acid (EPA, n-3), docosahexaenoic acid (DHA, n-3) and stearidonic acid (STA, n-3) wherein the weight ratio STA/DHA is at least 0.1 effectively reduces the reaction to the secrete of house dust mites, particularly dust mite induced respiratory insufficiency. The combination of animal and vegetable oil was found to be particularly effective. This is particularly advantageous because several advantages can be achieved by using vegetable oils, such as cost reduction and availability.

The composition is orally administered to a child with the age between 0 and 10 years.

Without wishing to be bound by theory, the following mechanism is believed to be responsible for the effectiveness of the present combination of fatty acids:
a. the inhibitory effect of STA, DGLA and EPA on the 5-lipoxygenase activity; and
b. the composition contains both high quantities of EPA and STA enabling an inhibiting effect of the delta-5 desaturase enzyme in the omega-6 synthetic pathway. This inhibition reduces the conversion of di-homo-gammalinolenic acid (DHGLA) to arachidonic acid (ARA), resulting in an accumulation of DHGLA. The accumulated DHGLA inhibits the dust mite induced reaction in the lungs, and thereby preventing respiratory insufficiency.
c. STA further in directly inhibits 5-lipoxygenase, which is responsible for the conversion of long chain polyunsaturated fatty acids into prostaglandins.

### DETAILED DESCRIPTION

The present invention provides a method for the treatment and/or prevention of dust mite induced respiratory insufficiency and/or dust mite allergy, said method comprising the administration of a pharmaceutical or nutritional composition comprising eicosapentaenoic acid (EPA, n-3), docosahexaenoic acid (DHA, n-3) and stearidonic acid (STA, n-3), said composition having a weight ratio STA /DHA of at least 0.1. The composition comprises fish oil and at least one stearidonic acid containing oil selected from the group consisting of borage oil and echium oil. The composition is orally administered to a child between 0 and 10 years

### LCPUFA

The present composition comprises eicosapentaenoic acid (EPA, n-3), docosahexaenoic acid (DHA, n-3) and stearidonic acid (STA, n-3), wherein the weight ratio STA/DHA is at least 0.1, preferably at least 0.2, even more at least 0.3. Preferably the weight ratio STA/DHA is below about 5, even more preferably below about 2, more preferably below about 1. A preferred range for the weight ratio STA/DHA is therefore 1 to 0.1. Compositions with this ratio STA/DHA are particularly effective.

The present composition has a weight ratio EPA/STA of at least 0.1, preferably of at least 1, even more of at least 5. Preferably the weight ratio EPA/STA is below about 25, even more preferably below about 10. Compositions with this ratio STA/DHA are particularly effective and indicative for the proper mixture of EPA containing (fish) and STA containing (vegetable) oil.

In a further preferred embodiment, the present invention comprises docosapentaenoic acid (DPA; 22:5n3), for a further increase of omega-3 fatty acids concentration and enable further reduce dust mite induced inflammatory reactions.

The present method preferably comprises the administration of at least 50 mg EPA per day, more preferably at least 100 mg EPA per day, even more preferably at least 250 mg EPA per day. Preferably the present method does not provide more than 10 g EPA per day. The present method preferably provides at least 10 mg STA per day, more preferably at least 25 mg STA per day, even more preferably at least 50 mg STA per day. Preferably the present method does not provide more than 5 g STA per day. The present method preferably provides at least 25 mg DHA per day, more preferably at least 50 mg DHA per day, even more preferably at least 100 mg DHA per day. Preferably the present method does not provide more than 10 g DHA per day. The present method preferably provides at least 10 mg DPA per day, more preferably at least 25 mg DPA per day, even more preferably at least 50 mg DPA per day.

To reduce the proinflammatory action as much as possible, the present composition is preferably low in arachidonic acid (ARA, n-6). Preferably the at least one, more preferably all of the weight ratios ARA/EPA, ARA/DHA, ARA/STA are all below 2, more preferably below 1, even more preferably all below 0.5. The present method preferably provides not more than 100 mg ARA per day, more preferably not more than 50 mg ARA per day, even more preferably not more than at least 25 mg ARA per day.

The present polyunsaturated fatty acids DHA, EPA, STA and/or DPA are preferably provided as free fatty acids, triglycerides and/or phospholipids. Preferably the present composition preferably comprises at least one of DHA, EPA, STA and/or DPA in triglyceride form.

The present composition contains fish oil and at least one STA containing oils selected from the group consisting of borage oil and echium oil.

The present composition is preferably produced by using sources of non human origin.

### Dust mite induced respiratory insufficiency

The present invention provides a method for the treatment and/or prevention of dust mite allergy, particularly dust mite induced respiratory insufficiency, particularly dust mite secrete induced respiratory insufficiency. Especially dust mite induced asthma (immediate phase with bronchoconstriction, to chronic state with bronchial eosinophiles infiltration and hypersecretion) and dust mite induced inflammatory diseases of the lung (e.g. dust mite induced rhinitis) can be treated and/or prevented. Dust mite induced diseases, e.g. dust mite induced respiratory insufficiency, is sometimes also referred to as dust induced allergic reaction. Dust mite induced respiratory insufficiencies which can be suitably treated and/or prevented include dust mite induced difficulty in breathing, dust mite induced heavy breathing, dust mite induced respiratory tract infection, dust mite induced irritation in the lungs, dust mite induced congestion in the lungs, dust mite induced excessive mucus production, dust mite induced breathlessness, dust mite induced bronchitis, dust mite induced bronchiolitis, dust mite induced tracheitis, dust mite induced pneumonia, dust mite induced sinusinitis, dust mite induced airflow obstruction and/or dust mite induced rhinitis.

### Subjects

The present method is particularly suitable for treatment and/or prevention of dust mite allergy in children with the age between 0 and 10 years, preferably infants with the age between 0 and 4 years.

Additionally, the present method can be advantageously used by patients likely to develop a respiratory tract infections disease, particularly patients infected with human immunodeficiency virus (HIV) and/or persons suffering from one or more of the following diseases: nephrotic syndrome, multiple myeloma, lymphoma, Hodgkin's disease, persons which have undergone organ transplantation, persons with chronic illnesses of the hart, kidney or lungs (especially chronic obstructive pulmonary disease (COPD), lung emphysema, sarcoidosis, cystic fybrosis, bronchiectasis, lung cancer, atelectasis, respiratory failure, occupational lung diseases, asthma), diabetes and alcoholism. The present method is advantageously used by patients with COPD, HIV infection and/or diabetes, as these patients are often weakened by the disease and likely to develop respiratory diseases.

In a further preferred embodiment, the present method comprises the administration of the present composition to patients, particularly patients that are on a ventilator or artificial breathing machine and patients in the intensive care unit. These patients are particularly vulnerable for inflammatory diseases and infections.

### Mode of administration

The composition used in the present method is preferably administered enterally, more preferably orally.

In a preferred embodiment the present composition is administered as a nutritional matrix, preferably containing a lipid, a protein and a carbohydrate component. This is particularly critical for infants who cannot yet swallow capsules. Hence, for infant with the age between 0 and 4 years, preferably the present fatty acids are embedded in a nutritional composition containing fat, carbohydrates and protein. In a preferred embodiment the present method comprises the administration of a nutritional composition which fulfills the requirements for feeding infants, particularly nutritional compositions containing between 10 and 60 en-% lipid, between 5 and 50 en-% protein, between 15 and 90 en-% carbohydrate. More preferably the nutritional composition contains between 7.5 to 12.5 energy-% protein; 40 to 55 energy-% carbohydrates; and 35 to 50 energy-% fat. (en-% is short for energy percentage and represents the relative amount each constituent contributes to the total caloric value of the preparation). In the present nutritional composition, preferably the present composition comprises at least 0.1 wt.-%, preferably at least 0.25 wt.-%, more preferably at least 0.5 wt.-%, even more preferably at least 0.75 wt.-% LC-PUFA with 20 and 22 carbon atoms of the total fat content. The content of LC-PUFA with 20 and 22 carbon atoms in the present composition, preferably does not exceed 15 wt.-% of the total fat content, preferably does not exceed 10 wt.-%, even more preferably does not exceed 5 wt.-% of the total fat content.

According to a further preferred embodiment, the present composition is ingested in the form of a capsule, pill or tablet (hereinafter unit dosage). This has the advantage that the diet of the subject does not have to be changed, and the fatty acid supplement can be ingested in addition to the normal diet. The unit dosage preferably has a weight between 0.1 and 3 grams per unit. The unit dosages preferably contains at least 50 wt.% fatty acids based on total weight of the unit dosage, preferably at least 75 wt.%. The fatty acids are preferably provided as free fatty acids, triglycerides and/or phospholipids. Preferably the present composition preferably comprises at least one of DHA, EPA, STA and/or DPA in triglyceride form.

### EXAMPLES

### Example 1: Clinical study

### Clinical trial

A placebo controlled, double blind, randomized study with parallel design was conducted to evaluate the effect of the ingestion of the present composition on the outcome and severity of dust mite secrete induced respiratory insufficiency.

### Study population

30 children with episodic asthma (GINA I) and dust mite allergy were recruited. Inclusion criteria for study participation were: young, mono-allergenic dust mite allergic patients with beginning asthma, absence of acute exacerbation of diseases, and written informed consent of the patients. Exclusion criteria were: exceptional para-clinical and clinical symptoms, no steady physical conditions, administration of anti-inflammatory drugs (e.g. corticosteroids, acetylsalicylic acid, NSAID etc.), and discontinuation of the supplementation for longer than two days more often than twice.

### Study design

An explorative, double blind study with parallel group design was conducted. A total of 30 young patients suffering from beginning asthma were enrolled in the study. The diet was not influenced by the protocol. Patients who enter the study were randomly assigned to one of the two supplementation groups.

The last day before start of the supplementation period was defined as examination day 1 (d1), the last day of a three week supplementation period was defined as examination day 2, and the last day of the continued supplementation period of 2 weeks was defined as examination day 3. Allergen stimulations were conducted at d1 (basic value) and three times between day 2 and 3.

During the screening visits (1 day, week 4 and 5), blood fatty acid pattern (erythrocytes /plasma), plasma eicosanoids (cys LT), sputum eosinophilic cells, eosinophilic cationic protein (ECP), plasma basophiles ex vivo stimulation with specific antigen and nitric oxide exhale (NO-exhale) were determined.

### Test products

The treatment and control groups received an equal amount of 3500 mg total fat/day (hereinafter supplement) in addition to the daily diet. The treatment group received a mixture of fish oil (Incromega™) (28.2 g/100 ml); sunflower oil (Trisun 80™, 11.6 g/100 ml; linseed oil (12.9 g/100 ml), borage oil (13.6 g/100 ml), milk fat (24.6 g/100 ml) and maize oil (9.0 g/100ml). The placebo group received a combination of canola oil, milk fat (75.3 g/100 ml); and maize oil (18.8 g/100 ml) (see Table 1). The fatty acid composition of the supplements received is given in Table 2.

**TABLE 1**

| **Oil** | **Treatment group** | **Placebo** |
|---|---|---|
| fish | 28,22 | |
| canola | | 5,91 |
| sunflower | 11,65 | |
| linseed | 12,95 | |
| milk | 24,58 | 75,29 |
| maize | 9,03 | 18,8 |
| borage | 13,57 | |
| percentage | 100 | 100 |

**TABLE 2**

| **Fatty acid** | **Treatment group** | **Control group** |
|---|---|---|
| C-16:0 | 9,03 | 23,61 |
| C-17:0 | 0,01 | 0,02 |
| C-18:0 | 3,48 | 7,56 |
| C-18:1w9 | 12,86 | 0 |
| C18:1w7+9 | 7,82 | 22,51 |
| C-18:2w6 | 13,03 | 12,80 |
| C-18:3w3 | 3,93 | 0,95 |
| C-18:3w6 | 1,92 | 0,03 |
| C-18:4w3 (STA) | 2,44 | 0 |
| C-20:4w6 (ARA) | 0,43 | 0 |
| C-20:5w3 (EPA) | 14,99 | 0 |
| C-22:5w3 (DPA) | 2,30 | 0 |
| C-22:6w3 (DHA) | 5,78 | 0 |

### Biochemical measurements

Fatty acids were analyzed by CGC-MS, eicosanoids were measured by ELISA after whole blood ex vivo stimulation, inflammatory parameters ex vivo are analyzed by standard in vitro bioassays.

### Statistical analysis

ANOVA/T-tests for parametric group tests of interval data; Kruskal-Wallis/U-tests for non-parametric group tests of interval data and ordinal data; Chi²-/Fisher's exact tests for qualitative (categorical and nominal) data; Kaplan-Meyer-curves/Log-rank tests for time-related data; multivariate analyses, regressions, logistic regressions for relationships between and interactions of parameters. Statistical differences were assumed when p < 0.05.

### Results

**CysLT:** Based on a significant change of the fatty acid composition of blood plasma and erythrocytes (compliance control) the eicosanoid pattern after whole blood *ex vivo* stimulation has been changed. The arachidonic acid dependent, pro-inflammatory cysteinyl leucotriene (cysLT) secretion after stimulation with allergen was decreased in the treatment group compared to the control group after 4 weeks administration of the present composition (see Table 1). Patients with asthma and respiratory disease have significantly higher cysteinyl leukotriene levels than control subjects, and the levels were significantly greater in asthmatic subjects as the level of disease severity increased (Pavord ID et al Am J Respir Crit Care Med. 1999;160:1905-1909).

The present inventors have found that by administering the present composition, the cysLT is statistically significant (p<0.05) reduced compared to control at visit 3, when stimulated with 2 ng/ml allergen (see Table 3). These results are indicative for the advantageous effects of the present invention, e.g. the treatment and/or prevention of the dust mite induced respiratory insufficiency.

**TABEL 3: CysLT**

| | **Visit 1** | **Visit 2** | **Visit 3** |
|---|---|---|---|
| **Placebo** | 1924 | 1590 | 2889 |
| **treatment** | 1512 | 1561 | 1119 |

**Eosinophilic cells:** The value for sputum eosinophilic cells per 400 cells is indicative for the severity of bronchio asthma and is a marker for the switch from the late phase response to chronic response. A higher value for sputum eosinophilic cells per 400 cells correlates with increased inflammation induced airflow limitations. The present results show a statistically significant reduction (p<0.05) in sputum eosinophilic cells per 400 cells for the treatment group (see Table 4). These results are indicative for the advantageous effects of the claimed invention.

**TABEL 4: Sputum eosinophilic cells per 400 cells**

| | **Visit 1** | **Visit 14** |
|---|---|---|
| **Placebo** | 6.0 | 35.6 |
| **Treatment** | 5.6 | 12.1 |

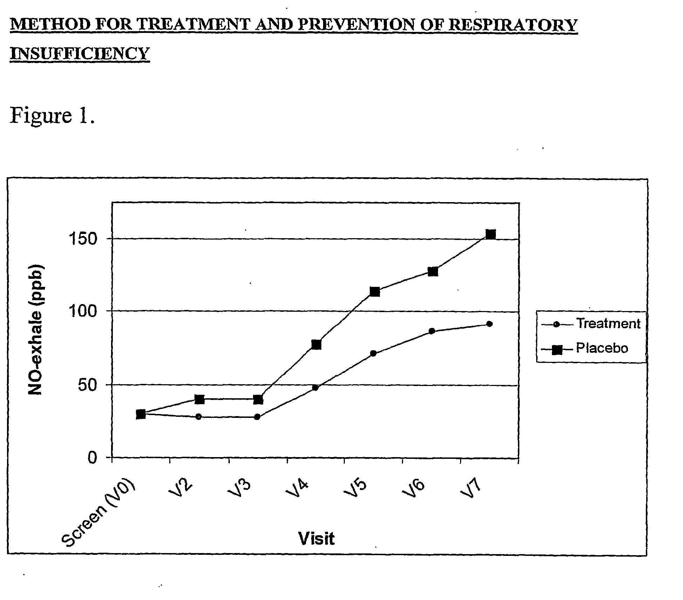

**NO-exhale:** The value for Nitric Oxide exhale (NO-exhale) is indicative for the severity of dust mite induced inflammatory response. The present data show that the NO-exhale is reduced at visit 6 and 7 (p<0.05) in the treatment group compared to the control group (see Table 5). Figure 1 shows the average NO-exhale of the treatment and placebo group during each visits 1-7. These results are indicative for the dust mite respiratory insufficiency reducing properties or the present composition.

**TABEL 5: NO exhale**

| | **Visit 0** | **Visit 6** | **Visit 7** |
|---|---|---|---|
| Placebo | 29.8 | 128.4 | 153.7 |
| Treatment | 30.2 | 86.1 | 91.3 |

**ECP:** When activated eosinophils release a number of substances, including the protein eosinophil cationic protein (ECP), which is antibiotic and cytotoxic. High ECP concentrations signify inflammation in the lung. Measurement of ECP levels in blood serum can be used to objectively measure the level of ECP, which in turn is closely related to the degree of inflammation in the lungs. In present results show that in the placebo group, the ECP level as statistically significant increased compared to treatment group when the difference in ECP between visit 1 and visit 8 are compared (V1-V8) (see, Table 6). These results are indicative for the advantageous effects of the claimed invention.

**TABEL 6: Plasma ECP**

| | **V2-V1** | **V8-V1** |
|---|---|---|
| **Placebo** | -4.7 | 20.5 |
| **Treatment** | -13 | -1.7 |

### Example 2: Infant nutrition

Infant nutrition with a label containing an indication that it can be suitably used for the prevention of dust mite secrete induced respiratory insufficiency.

Energy: 66 kcal; Protein: 8 en% ; Digestible Carbohydrates: 44 en% (containing 7.3 g lactose); Fibre: 0.8 g (containing 0.05 g fructopolysaccharide (Raftiline HP™, Orafti, Tienen, Belgium); 0.55 g transgalactooligosaccharides (Vivinal-GOS^{™} (Borculo Domo Ingredients, Netherlands); Lipid: 48 en-% (containing, based on total weight of the lipid 0.2 wt.-% DHA; 0.07 wt.-% EPA; 0.5 wt.-% STA; 2.2 wt.-% ALA, 0.2 wt.% GLA; 13 wt.-% LA)
; 0.20 g pectin hydrolysate prepared as described in EP-A 1 373 543, example 1. Osmolarity: 300 mOsmol/l. The composition further contains choline (6 mg/100 ml) and taurine (6.3 mg/100 ml); minerals and trace elements (including 2 mg zinc/100 ml) and vitamins in amounts in compliance with the international guidelines for infant milk formula.

## Claims

1. Use of a composition comprising eicosapentaenoic acid (EPA, n-3), docosahexaenoic acid (DHA, n-3) and stearidonic acid (STA) and having a weight ratio STA/DHA of at least 0.1 for the manufacture of a pharmaceutical or nutritional composition for the treatment and/or prevention of dust mite allergy wherein the composition comprises fish oil and at least one stearidonic acid containing oil selected from the group consisting of borage oil and echium oil and wherein the pharmaceutical or nutritional composition is orally administered to a child with the age between 0 and 10 years

2. Use according to claim 1, comprising the administration of between 50 mg and 10 g EPA per day, between 10 mg and 5 gram STA and between 25 mg and 10 g DHA per day.

3. Use according to claim 1 or 2, comprising the administration of at least 10 mg docosapentaenoic acid (DPA) per day.

4. Use according to any one of the preceding claims comprising the administration of not more than 100 mg arachidonic acid (ARA) per day.

5. Use according to any one of the preceding claims wherein the composition comprises between 10 and 60 en-% lipid, between 5 and 50 en-% protein and between 15 and 90 en-% carbohydrate.

6. Use according to any one of the preceding claims, wherein the pharmaceutical or nutritional composition is administered in the form of a capsule, pill or tablet.

7. A pharmaceutical or nutritional composition comprising eicosapentaenoic acid (EPA, n-3), docosahexaenoic acid (DHA, n-3) and stearidonic acid (STA) and having a weight ratio STA/DHA of at least 0.1 and comprising fish oil and at least one stearidonic acid containing oil selected from the group consisting of borage oil and echium oil for use in the treatment and/or prevention of dust mite allergy wherein the composition is orally administered to a child with the age between 0 and 10 years.

8. Composition according to claim 7 comprising the administration of between 50 mg and 10 g EPA per day, between 10 mg and 5 gram STA and between 25 mg and 10 g DHA per day.

9. Composition according to claim 7 or 8 comprising the administration of at least 10 mg docosapentaenoic acid (DPA) per day.

10. Composition according to anyone of the claims7 to 9 comprising the administration of not more than 100 mg arachidonic acid (ARA) per day.

11. Composition according to anyone of the claims7 to 10 comprising between 10 and 60 en-% lipid, between 5 and 50 en-% protein and between 15 and 90 en-% carbohydrate.

12. Composition according to anyone of the claims7 to 11 in the form of a capsule, pill or tablet.

## Patentansprüche

1. Verwendung einer Zusammensetzung umfassend Eicosapentaensäure (EPA, n-3), Docosahexaensäure (DHA, n-3) und Stearidonsäure (STA) mit einem Gewichtsverhältnis STA/DHA von mindestens 0,1 für die Herstellung einer pharmazeutischen Zusammensetzung oder einer Nahrungszusammensetzung für die Behandlung und/oder Verhinderung von Staubmilbenallergie, wobei die Zusammensetzung Fischöl und mindestens ein Stearidonsäure enthaltendes Öl, das ausgewählt ist aus der Gruppe bestehend aus Borretschöl und Echiumöl, umfasst und wobei die pharmazeutische Zusammensetzung oder Nahrungszusammensetzung oral an ein Kind im Alter zwischen 0 und 10 Jahren verabreicht wird.

2. Verwendung nach Anspruch 1, umfassend die Verabreichung von zwischen 50 mg und 10 g EPA pro Tag, zwischen 10 mg und 5 g STA und zwischen 25 mg und 10 g DHA pro Tag.

3. Verwendung nach Anspruch 1 oder 2, umfassend die Verabreichung von mindesten 10 mg Docosapentaensäure (DPA) pro Tag.

4. Verwendung nach einem der vorhergehenden Ansprüche umfassend die Verabreichung von nicht mehr als 100 mg Arachidonsäure (ARA) pro Tag.

5. Verwendung nach einem der vorhergehenden Ansprüche, wobei die Zusammensetzung zwischen 10 und 60 en-% Lipid, zwischen 5 und 50 en-% Protein und zwischen 15 und 90 en-% Kohlenhydrat umfasst.

6. Verwendung nach einem der vorhergehenden Ansprüche, wobei die pharmazeutische Zusammensetzung oder die Nahrungszusammensetzung in Form einer Kapsel, Pille oder Tablette verabreicht wird.

7. Pharmazeutische Zusammensetzung oder Nahrungszusammensetzung umfassend Eicosapentaensäure (EPA, n-3), Docosahexaensäure (DHA, n-3) und Stearidonsäure (STA) mit einem Gewichtsverhältnis STA/DHA von mindestens 0,1 und umfassend Fischöl und mindestens ein stearindonsäurehaltiges Öl ausgewählt aus der Gruppe bestehend aus Borretschöl und Echiumöl zur Verwendung bei der Behandlung und/oder Verhinderung von Staubmilbenallergie, wobei die Zusammensetzung oral an ein Kind im Alter zwischen 0 und 10 Jahren verabreicht wird.

8. Zusammensetzung nach Anspruch 7 umfassend die Verabreichung von zwischen 50 mg und 10 g EPA pro Tag, zwischen 10 mg und 5 g STA und zwischen 25 mg und 10 g DHA pro Tag.

9. Zusammensetzung nach Anspruch 7 oder 8 umfassend die Verabreichung von mindestens 10 mg Docosapentaensäure (DPA) pro Tag.

10. Zusammensetzung nach einem der Ansprüche 7 bis 9 umfassend die Verabreichung von nicht mehr als 100 mg Arachidonsäure (ARA) pro Tag.

11. Zusammensetzung nach einem der Ansprüche 7 bis 10 umfassend zwischen 10 und 60 en-% Lipid, zwischen 5 und 50 en-% Protein und zwischen 15 und 90 en-% Kohlenhydrat

12. Zusammensetzung nach einem der Ansprüche 7 bis 11 in Form einer Kapsel, Pille oder Tablette.

## Revendications

1. Utilisation d'une composition comprenant de l'acide éicosapentaenoïque (EPA, n-3), de l'acide docosahexaenoïque (DHA, n-3) et de l'acide stéaridonique (STA) et ayant un rapport massique STA/DHA d'au moins 0,1 pour la fabrication d'une composition pharmaceutique ou nutritionnelle pour le traitement et/ou la prévention de l'allergie aux acariens, la composition comprenant de l'huile de poisson et au moins un acide stéaridonique contenant de l'huile sélectionnée dans le groupe constitué de l'huile de bourrache et de l'huile d'échium, et la composition pharmaceutique ou nutritionnelle étant administrée par voie orale à un enfant âgé de 0 à 10 ans.

2. Utilisation selon la revendication 1, comprenant l'administration de entre 50 mg et 10 g d'EPA par jour, entre 10 mg et 5 g de STA et entre 25 mg et 10 g de DHA par jour.

3. Utilisation selon la revendication 1 ou 2, comprenant l'administration d'au moins 10 mg d'acide docosapentaenoïque (DPA) par jour.

4. Utilisation selon l'une quelconque des revendications précédentes, comprenant l'administration de pas plus de 100 mg d'acide arachidonique (ARA) par jour.

5. Utilisation selon l'une quelconque des revendications précédentes, la composition comprenant entre 10 et 60 en-% de lipide, entre 5 et 50 en-% de protéine et entre 15 et 90 en-% de carbohydrate.

6. Utilisation selon l'une quelconque des revendications précédentes, où la composition pharmaceutique ou nutritionnelle est administrée sous la forme d'une gélule, d'une pillule ou d'un comprimé.

7. Composition pharmaceutique ou nutritionnelle comprenant de l'acide éicosapentaenoïque (EPA, n-3), de l'acide docosahexaenoïque (DHA, n-3) et de l'acide stéaridonique (STA) et ayant un rapport massique STA/DHA d'au moins 0,1 et comprenant de l'huile de poisson et au moins un acide stéaridonique contenant de l'huile sélectionnée dans le groupe constitué de l'huile de bourrache et de l'huile d'échium, destinée à être utilisée dans le traitement et/ou la prévention de l'allergie aux acariens, la composition étant administrée par voie orale à un enfant âgé de 0 à 10 ans.

8. Composition selon la revendication 7, comprenant l'administration de entre 50 mg et 10 g d'EPA par jour, entre 10 mg et 5 g de STA et entre 25 mg et 10 g de DHA par jour.

9. Composition selon la revendication 7 ou 8, comprenant l'administration d'au moins 10 mg d'acide docosapentaenoïque (DPA) par jour.

10. Composition selon l'une quelconque des revendications 7 à 9, comprenant l'administration de pas plus de 100 mg d'acide arachidonique (ARA) par jour.

11. Composition selon l'une quelconque des revendications 7 à 10, comprenant entre 10 et 60 en-% de lipide, entre 5 et 50 en-% de protéine et entre 15 et 90 en-% de carbohydrate.

12. Composition selon l'une quelconque des revendications 7 à 11 sous la forme d'une gélule, d'une pillule ou d'un comprimé.
